# FASCICULE DE BREVET EUROPEEN

(11) **EP 1 129 714 B1**
(45) Date de publication et mention de la délivrance du brevet: **12.10.2005**
(21) Numéro de dépôt: 01400068.1
(22) Date de dépôt: 11.01.2001
(51) Int. Cl.: A61K 31/663, A61P 19/10

(54) **Utilisation de l'acide tiludronique et de ses dérivés chez la volaille pour la préparation d'un médicament destiné à prévenir et à traiter l'ostéoporose**
Verwendung von Tiludronsäure beim Geflügel zur Herstellung eines Arzneimittels zur Prophylaxe und Behandlung von Osteoporose
Use of tiludronic acid and its derivatives in poultry for the preparation of a medicine for preventing and treating osteoporosis

(30) Priorité: 12.01.2000 FR 0000356
(43) Date de publication de la demande: 05.09.2001
(73) Titulaire: CEVA SANTE ANIMALE, 33500 Libourne (FR)
(72) Inventeur: Bardon, Thierry, 33270 Bouliac (FR); Thibaud, Dominique, 33470 Gujan Mestras (FR)
(74) Mandataire: Bernasconi, Jean Raymond

(56) Documents cités:
- EP-A- 0 950 417
- WO-A-00/64516
- WO-A-94/14455
- WILSON S ET AL: "Bisphosphonates: a potential role in the prevention of osteoporosis in laying hens." RESEARCH IN VETERINARY SCIENCE, (1998 JAN-FEB) 64 (1) 37-40., XP000949202
- THORP B H; WILSON SANDRA; RENNIE SARAH; SOLOMON SALLY E: "The effect of a bisphosphonate on bone volume and eggshell structure in the hen." AVIAN PATHOLOGY, vol. 22, no. 4, 1993, pages 671-682, XP000949321
- CHESTNUT C H III: "Tiludronate: Development as an osteoporosis therapy." BONE (NEW YORK), vol. 17, no. 5 SUPPL., 1995, pages 517S-519S, XP000938252
- KLEIN L ET AL: "Effects of ethane-1-hydroxy-1,1- diphosphonate ( EHDP ) upon the kinetics of bone resorption and bone formation at the whole bone level in prelabelled chicks." CALCIFIED TISSUE INTERNATIONAL, (1983 JUL) 35 (4-5) 602-8., XP000938338
- DAVID PE'ER; NGUYEN HIEU; BARBIER ALAIN; BARON ROLAND: "The bisphosphonate tiludronate is a potent inhibitor of the osteoclast vacuolar H+-ATPase." JOURNAL OF BONE AND MINERAL RESEARCH, vol. 11, no. 10, 1996, pages 1498-1507, XP000938272

## Description

L'invention concerne l'utilisation de l'acide tiludronique, d'un de ses sels pharmaceutiquement acceptables ou d'un de ses hydrates dans la préparation d'un médicament destiné à prévenir et à traiter l'ostéoporose chez la poule pondeuse.

Les oiseaux femelles ont un métabolisme osseux particulièrement sollicité dans le but de produire la coquille des oeufs riche en minéraux, au premier rang desquels le calcium. L'os chez les oiseaux femelles remplit 2 fonctions essentielles : une fonction mécanique, commune à l'ensemble des vertébrés, conférant les propriétés de maintien du corps dans l'espace et une fonction physiologique, propre aux oiseaux femelles, en constituant une réserve calcique facilement mobilisable en vue de la production de la coquille de l'oeuf. La fonction mécanique est assurée par 2 types d'os : l'os cortical constituant l'enveloppe externe des os et l'os trabéculaire, constituant des travées osseuses orientées selon les lignes de force à l'intérieur des os. La fonction physiologique pour l'essentiel est assurée par un troisième type d'os, l'os médullaire qui occupe les cavités de certains os longs ou d'os plats. L'os médullaire se développe entre les travées de l'os trabéculaire. Cet os très fragile n'assure aucune fonction mécanique.

En période de ponte, l'os médullaire subit des cycles de résorption - formation, chaque cycle accompagnant la formation d'un oeuf. En se résorbant, l'os médullaire libère une quantité importante de calcium nécessaire à la production de la coquille de l'oeuf. La formation qui accompagne cette résorption permet la reconstitution de la réserve calcique médullaire qui sera mobilisée au cycle suivant.

Parmi les oiseaux femelles, la poule, en particulier la poule pondeuse d'oeufs destinés à la consommation humaine, est l'espèce pour laquelle le métabolisme osseux est fortement sollicité. En effet, les progrès constants dans les domaines de la sélection génétique et de la nutrition ont conduit au cours des 30 dernières années à une augmentation régulière du nombre d'oeufs pondus par poule. Il est fréquent désormais qu'une poule ponde plus de 300 oeufs sur une année de ponte. Au plus fort de la phase productive, une poule pond un oeuf par jour. Paradoxalement, cette évolution s'est accompagnée d'une réduction de la taille des animaux et donc, en particulier, du volume de la réserve calcique que représente le squelette.

Le métabolisme osseux chez la poule n'a pas encore été étudié de façon exhaustive. Cependant, les modifications subies par l'os médullaire et l'os structural en relation avec la ponte commencent à être mieux comprises (voir par exemple les articles de Wilson S. et al, 1992, *Res. Vet. Sci*., **53,** 52-58, de Miller S.C., p. 103-116 et de Whitehead C.C. et al, p. 265-280 publiés dans le livre « Bone biology and skeletal disorders in poultry », 1992, Ed. C.C. Whitehead, Carfax Publishing Company). Au fur et à mesure que progresse la ponte, le volume d'os médullaire augmente de façon à assurer la production de la coquille des oeufs et le maintien de cette production au cours du temps. Le calcium stocké dans l'os médullaire a pour principale origine le calcium alimentaire. Mais l'os structural participe également à la constitution de la réserve calcique médullaire. Ainsi, progressivement au cours de la ponte, alors que l'os médullaire subit un remodelage permanent à chaque cycle de ponte, l'os structural (os cortical et os trabéculaire) subit une résorption libérant le calcium qui va ensuite être stocké dans l'os médullaire. Cette résorption n'est pas compensée par la formation d'un nouvel os structural. La résultante de ce phénomène est une perte d'os structural au cours de la ponte sans que la minéralisation osseuse en soit affectée. Ceci caractérise l'ostéoporose de la poule. Ce processus progressif de perte osseuse est précédée d'une autre perte d'os structural accompagnant cette fois-ci la formation de l'os médullaire, formation qui débute avec le développement des follicules ovariens environ 2 semaines avant l'entrée en ponte (Wilson S. et al, Res. *Vet. Sci*., 1998, **64,** 37-40). Ainsi, l'ostéoporose débute précocément chez la poule pondeuse, au moment de l'acquisition de la maturité sexuelle : la perte d'os structural à ce stade accompagne la formation de l'os médullaire. Elle se poursuit tout au long de la ponte : elle accompagne alors le remodelage de l'os médullaire indispensable à la production de la coquille de l'oeuf.

Les conséquences de l'ostéoporose chez la poule sont de divers ordres : médical, bien-être et économique. En effet, la perte de substance osseuse corticale et trabéculaire est à l'origine d'une fragilisation du squelette conduisant à l'apparition de fractures spontanées en cours de ponte ou de fractures provoquées par la manipulation des animaux en fin de ponte lors de la conduite à l'abattoir. Des troubles locomoteurs sont associés à la fragilisation du squelette, au point de réduire voire de supprimer les déplacements de l'animal qui finit par ne plus s'alimenter et mourrir. Le bien-être de l'animal est ainsi remis en cause (douleur des fractures, déplacements limités, mauvaise alimentation). Enfin, au plan économique, l'ostéoporose est génératrice de pertes liées à la mortalité, à la baisse de la production d'oeufs et à la non-valeur économique des carcasses des poules porteuses de fractures.

Par les conséquences qu'elle entraine, l'ostéoporose est une des préoccupations majeures de l'industrie de l'élevage de la poule pondeuse d'oeufs destinés à la consommation humaine (Knowles T.G. et al, 1998, *Poultry Science,* **77,** 1798-1802). La recherche de solutions permettant de réduire l'importance du processus ostéoporotique chez la poule pondeuse répond à un besoin fort de cette industrie.

Les dérivés de l'acide bisphosphonique, ou bisphosphonates, d'intérêt médical sont aujourd'hui bien connus. Leurs propriétés pharmacologiques et leurs applications thérapeutiques sont bien décrites dans l'état de l'art chez les mammifères, chez l'homme en particulier. Les dérivés de l'acide bisphosphonique exercent des propriétés anti-résorptives sur l'os et une action régulatrice du remodelage osseux. La principale cible cellulaire de ces composés est l'ostéoclaste, cellule responsable de la résorption osseuse. Le mécanisme d'action cellulaire est en voie d'élucidation. Il semble désormais qu'au sein de la famille des bisphosphonates il soit possible de distinguer 2 groupes de composés agissant de façon distincte sur le métabolisme de l'ostéoclaste : les composés dépourvus d'une fonction azotée, fonction amine en particulier, et les composés possédant une telle fonction. Dans le premier groupe, on trouve des composés tels que l'acide étidronique, l'acide clodronique ou l'acide tiludronique. Ces composés exercent en partie leur action cytotoxique sur les ostéoclastes au travers de métabolites formés au sein de la cellule ; ces métabolites sont des nucléotides non hydrolysables analogues de l'ATP (Auriola S. et al., 1997, *J. Chromat. B,* **704**, 187-195, Rogers M.J. et al, 1999, *Bone,* **24,** 73S-79S). Dans le second groupe (bisphosphonates à fonction azotée), on trouve des composés tels que l'acide pamidronique, l'acide alendronique, l'acide risédronique, l'acide ibandronique. Ces composés exercent une action cytotoxique au travers de l'inhibition de la voie métabolique du mévalonate conduisant à l'absence d'activation de protéines nécessaires à l'action ostéolytique des ostéoclastes (Luckman S.P., 1998, *J. Bone Miner. Res*., **13,** 581-589).

A ce mode d'action différent au niveau cellulaire correspond une différence d'intensité de l'effet pharmacologique sur la résorption osseuse. Les bisphosphonates non azotés apparaissent moins puissants que les composés azotés. A titre d'exemple, il est rapporté que par voie sous-cutanée la plus faible dose ayant entrainé un effet inhibiteur sur un modèle de résorption osseuse chez le rat est 300 à 400 fois plus faible avec l'acide alendronique qu'avec l'acide tiludronique (Geddes A., 1994, in « Bone and Mineral Research » Ed. Elsevier Science BV, p. 265-306).

Cette différence d'effet pharmacologique est retrouvée au plan clinique dans les indications pour lesquelles ces composés bénéficient d'autorisations de mise sur le marché en tant que médicaments. Parmi celles-ci, l'ostéoporose de la femme ménopausée est une indication privilégiée des dérivés de l'acide bisphosphonique. Certains dérivés ont une autorisation de mise sur le marché dans cette indication ; c'est le cas de l'acide alendronique, à la dose quotidienne de 10 mg par personne par voie orale (Lourwood D.L., 1998, *Pharmacotherapy*, **18,** 779-789). D'autres composés n'ont pas fait la preuve de leur efficacité dans cette indication. C'est le cas de l'acide tiludronique à la dose de 50 ou 200 mg par personne par voie orale (Reginster J.Y., 1998, *Bone,* **23** (5) Suppl., S594). Il est à noter que le rapport des doses quotidiennes entre les 2 composés dans cette indication (ostéoporose) est de 5 à 20, soit très inférieur au rapport des doses précédemment rappelé entre ces 2 mêmes composés sur un modèle d'inhibition de résorption.

WO0064516 citée au titre de l'article 54(3) CBE divulgue l'administration parentérale contrôlée de bisphosphonates, comme par exemple le tiludronate, pour le traitement de l'ostéoporose chez les animaux, par exemple la dinde, et chez l'homme.

WO9414455 décrit l'utilisation d'une combinaison d'un oestrogène et un bisphosphonate, par exemple le tiludronate, pour l'inhibition de la résorption osseuse, la prévention de la perte osseuse et la promotion de la formation osseuse chez les animaux, comme par exemple les oiseaux, et chez l'homme, souffrant d'ostéoporose.

EP0950417 rapporte l'utilisation d'une combinaison de la leptine avec un bisphosphonate, comme l'acide tiludronique ou sa forme ionique, pour le traitement de l'ostéoporose chez les animaux, par exemple la volaille, et chez l'homme.

Chez les oiseaux, peu de travaux ont été conduits à ce jour pour étudier les effets des dérivés de l'acide bisphosphonique dans le traitement et la prévention de l'ostéoporose. Une première étude démontre l'effet inhibiteur de l'acide alendronique sur la résorption de l'os trabéculaire qui accompagne l'acquisition de la maturité sexuelle chez la poule pondeuse lorsque le produit est administré par voie sous-cutanée à la dose de 0,01 mg/kg deux fois par semaine à partir de 16 semaines d'âge et jusqu'à la ponte du premier oeuf, date à laquelle les poules ont été sacrifiées afin de réaliser un examen histomorphométrique en région proximale de l'os tarsométatarse (Thorp B. H. et al, 1993, *Avian Pathol.,* **22,** 671-682). Une seconde étude utilise un protocole d'administration légèrement différent : l'acide alendronique est administré par la voie sous-cutanée également à la dose de 0,01 mg/kg à raison de 6 administrations au total réparties sur 2 semaines à partir de l'âge de 14 semaines, soit une dose totale de 0,06 mg/kg administrée à chaque poule (Wilson S. et al, *Res. Vet. Sci*., 1998, **64,** 37-40). Dans cette étude une moitié de l'effectif de poules témoins et traitées est sacrifiée à l'entrée en ponte, l'autre moitié étant sacrifiée environ 18 semaines après l'entrée en ponte. Comme la précédente, cette étude démontre l'effet inhibiteur de l'alendronate sur la résorption osseuse qui précède la ponte du premier oeuf. Dans les 2 cas, le volume osseux trabéculaire est de 25 à 30 % plus élevé en moyenne chez les poules traitées que chez les poules témoins. Après 18 semaines de ponte, les poules traitées ont toujours un volume osseux trabéculaire plus élevé d'environ 35 % que les poules témoins mais, dans les 2 groupes d'animaux, la perte osseuse trabéculaire signant l'évolution du processus ostéoporotique est comparable et représente environ 25 à 30 % du volume osseux trabéculaire à l'entrée en ponte. Ces deux études permettent donc de conclure que l'acide alendronique réduit la perte osseuse qui accompagne la formation de l'os médullaire avant l'entrée en ponte mais ne permet pas de réduire significativement la perte osseuse qui accompagne le remodelage de l'os médullaire en cours de ponte.

Ces travaux suggèrent donc qu'un dérivé d'acide bisphosphonique permet de prévenir partiellement l'ostéoporose de la poule pondeuse sans toutefois inhiber la résorption osseuse qui accompagne le remodelage de l'os médullaire en cours de ponte.

Ces travaux révèlent par ailleurs que l'acide alendronique entraine une réduction du volume de l'os médullaire non significative à l'entrée en ponte mais qui devient significative en cours de ponte : 18 semaines après l'entrée en ponte, les poules traitées par l'acide alendronique ont un volume osseux médullaire environ 20 % plus faible que celui des animaux témoins. Ce résultat est défavorable car il semble indiquer que l'administration de dérivés d'acide bisphosphonique peut avoir un impact sur la formation de la coquille des oeufs et, par voie de conséquence, sur la production d'oeufs. Les travaux publiés ne rapportent pas de résultats sur la production d'oeufs lors de traitement avant l'entrée en ponte. Par contre, l'étude de Thorp et al. démontre clairement un effet néfaste sur la production d'oeufs lorsque le bisphosphonate est administré en cours de ponte, avec une diminution voire un arrêt de ponte en fonction de la dose utilisée.

Ainsi, l'état de l'art conduit à penser que, si les dérivés de l'acide bisphosphonique, administrés avant l'entrée en ponte, sont potentiellement actifs pour traiter ou prévenir l'ostéoporose de la poule, ils agissent principalement sur l'inhibition partielle de la perte osseuse qui accompagne la formation de l'os médullaire avant l'entrée en ponte sans avoir d'effet sur la perte osseuse qui accompagne de remodelage de l'os médullaire en cours de ponte. Par ailleurs, cet effet inhibiteur de la perte osseuse est associé à un effet défavorable sur l'os médullaire susceptible de compromettre la production de coquilles d'oeufs de qualité, voire la production d'oeufs. Ceci remet en cause l'intérêt potentiel des bisphosphonates dans le traitement ou la prévention de l'ostéoporose de la poule ; il est essentiel en effet que le traitement préserve le potentiel de production d'oeufs pour être économiquement acceptable par un éleveur.

Par extrapolation, on aurait pu s'attendre à obtenir des effets analogues, et donc insatisfaisants, avec l'acide tiludronique à des doses plus élevées ( de 300 à 400 fois plus élevées) que celles utilisées dans les études réalisées avec l'acide alendronique, soit autour de 20 - 25 mg/kg in toto par voie sous-cutanée. Une comparaison des études réalisées chez l'homme avec l'acide tiludronique d'une part et avec l'acide alendronique d'autre part incite à penser en effet que des doses inférieures en acide tiludronique (doses suboptimales) sont insuffisantes pour obtenir des effets significatifs dans le traitement de l'ostéoporose post-ménopausale.

Or, de façon inattendue, les inventeurs ont démontré que des doses suboptimales d'acide tiludronique pouvaient prévenir l'ostéoporose chez la poule pondeuse, non seulement en inhibant la perte osseuse qui accompagne la formation d'os médullaire avant l'entrée en ponte mais aussi en inhibant la perte osseuse qui accompagne le remodelage de l'os médullaire en cours de ponte. Ce résultat est d'autant plus remarquable que l'acide tiludronique a été administré plusieurs semaines avant l'entrée en ponte ; les effets du produit sont encore mesurables plusieurs mois après la fin de son administration. Cet effet positif sur le traitement de l'ostéoporose ne s'accompagne pas d'un effet négatif sur l'os médullaire. L'acide tiludronique n'entraine pas de réduction du développement de l'os médullaire en cours de ponte. Les effets défavorables sur la production de coquilles de qualité ou sur la production d'oeufs observés par administration d'acide alendronique ne sont donc plus à craindre.

Plus précisément, l'invention concerne l'utilisation d'une substance active choisie parmi l'acide tiludronique, un de ses sels pharmaceutiquement acceptable, un de ses hydrates et leurs mélanges, dans la préparation d'un médicament destiné à prévenir et à traiter l'ostéoporose chez la poule pondeuse.

Par acide tiludronique, on entend le composé répondant à la formule suivante :

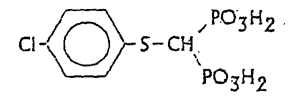

Les sels de ce composé avec des acides ou des bases minéraux ou organiques pharmaceutiquement acceptables sont également utilisables dans le cadre de l'invention. Des exemples de sels avec des acides sont les chlorhydrate, bromhydrate, sulfate, acétate, hydrogénosulfate, dihydrogénophosphate, méthanesulfonate, méthylsulfate, maléate, fumarate, sulfonate, 2-naphtalènesufonate, glycolate, gluconate, citrate, iséthionate, benzoate, salicylate, ascorbate, tartrate, succinate, lactate, glutarate, toluènesulfonate et ascorbate. Comme exemple de sels avec des bases minérales ou organiques, on peut citer les sels d'ammonium ou les sels de métaux alcalins, tels que par exemple les sels de sodium.

Les sels de sodium de l'acide tiludronique et notamment le sel disodique de celui-ci, sont plus particulièrement préférés à titre de substance active.

Les hydrates de ces composés sont de même utilisables selon l'invention.

La voie d'administration peut être la voie orale, la voie parentérale ou la voie nasale. Par voie orale, le traitement peut être administré dans l'eau de boisson. Par voie parentérale, on peut utiliser la voie sous-cutanée, intradermique, intramusculaire, intraveineuse ou intra-articulaire. Par voie nasale, le traitement peut être administré au moyen de dispositifs assurant la dispersion dans l'air de fines gouttelettes de liquide, préférentiellement d'eau, dans lequel aura été préalablement incorporé le médicament. De tels dispositifs sont par exemple des nébuliseurs, des atomiseurs, des vaporisateurs, des aérosols.

La forme galénique d'administration du médicament est fonction de la voie d'administration. Par la voie orale, des formes solubilisables dans l'eau de boisson sont préférées. Parmi celles-ci, on peut citer des poudres orales, des comprimés à dissolution rapide, des comprimés effervescents, des solutions buvables. Pour la voie parentérale, le traitement peut être administré sous la forme de solution, préférentiellement aqueuse, de suspension, d'implants ou de préparations lyophilisées.

Les préparations destinées à la voie orale peuvent contenir, en plus de l'acide tiludronique, un agent de désintégration, un agent d'écoulement, un lubrifiant et tout excipient de masse convenable.

Comme excipient de masse, on peut utiliser le lactose, la cellulose ou les amidons. Comme lubrifiant, on peut utiliser l'acide stéarique, le stéarate de magnésium, la L-leucine ou, par exemple, le tribéhénate de glycérol. Comme agent de désintégration, on peut utiliser le carboxyméthylamidon sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

La présente invention se réfère également aux formes orales à dissolution instantanée et aux formes orales effervescentes obtenues en ajoutant un couple effervescent à la composition selon l'invention. Comme couple effervescent, on peut utiliser, l'acide tartrique et le bicarbonate de sodium ou l'acide citrique et le bicarbonate de sodium.

L'invention se réfère également aux comprimés à dissolution instantanée, aux comprimés effervescents ainsi qu'aux comprimés recouverts d'un enrobage. Une composition contenant du laurylsulfate de sodium selon le brevet européen EP 336851 est particulièrement convenable.

Les préparations injectables sont préparées par mélange d'un ou plusieurs dérivés de l'acide bisphosphonique avec un régulateur de pH, un agent tampon, un agent de mise en suspension, un agent de solubilisation, un stabilisant, un agent de tonicité et/ou un conservateur, et par transformation du mélange en une injection intraveineuse, sous-cutanée, intramusculaire, intradermique ou intra-articulaire selon un procédé classique. Le cas échéant, les préparations injectables peuvent être lyophilisées selon un procédé classique.

Des exemples d'agents de mise en suspension englobent la méthylcellulose, le polysorbate 80, l'hydroxyéthylcellulose, l'acacia, la gomme adragante en poudre, la carboxyméthycellulose sodique et le monolaurate de sorbitane polyéthoxylé.

Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthylique d'acide gras d'huile de ricin.

En outre, le stabilisant englobe le sulfite de sodium, le métalsulfite de sodium et l'éther, tandis que le conservateur englobe le p-hydroxybenzoate de méthyle, le p-hydroxybenzoate d'éthyle, l'acide sorbique, le phénol, le crésol et le chlorocrésol.

Un exemple d'agent de tonicité est le mannitol.

Lors de la préparation des solutions ou suspensions injectables, il est souhaitable de veiller à ce qu'elles soient isotoniques au sang.

L'importance des effets obtenus est maximale lorsque l'acide tiludronique est administré avant l'entrée en ponte.

De manière avantageuse, l'administration d'acide tiludronique est initiée avant l'âge moyen d'entrée en ponte, dans les deux mois précédant l'entrée en ponte, de préférence les 6 semaines précédant l'entrée en ponte.

La durée de traitement est fonction de la voie d'administration. L'acide tiludronique peut être administré en une seule fois ou à intervalles (de préférence à intervalles réguliers) en l'espace de quelques jours ou de quelques semaines.

De manière préférée, l'administration d'acide tiludronique est stoppée dès l'entrée en ponte.

Le rythme d'administration doit également être choisi en fonction de la voie d'administration et de la dose.

L'administration peut être effectuée par voie nasale, orale ou parentérale.

Par voie orale ou par voie nasale, il est préféré d'administrer le traitement de façon répétée selon un rythme quotidien, biquotidien, hebdomadaire ou à intervalles réguliers tous les 2 à 6 jours. La dose par administration peut être donnée en une seule fois ou de façon fragmentée sur plusieurs heures ou encore de façon continue pendant une période de temps de quelques heures.

Par voie parentérale, le traitement peut être administré soit en injection unique, soit en injections répétées selon un rythme quotidien, biquotidien, hebdomadaire ou à intervalles réguliers tous les 2 à 6 jours. Un rythme préféré de traitement par voie parentérale est le traitement en injection unique.

Les doses recommandées varient en fonction de la voie d'administration. Par voie orale ou nasale, les doses par administration sont comprises entre 0,5 et 50 mg/kg, de façon préférée entre 1 et 20 mg/kg. Par voie parentérale, les doses par administration sont comprises 0,1 et 50 mg/kg, de façon préférée entre 0,25 et 25 mg/kg.

### Exemple

Un effectif de 216 poules de souche ISA Brown âgées de 15 à 17 semaines a été réparti en 3 groupes dans l'objectif d'évaluer les effets sur l'os d'une administration unique par voie sous-cutanée d'acide tiludronique, sous la forme de son sel de sodium (tiludronate disodique).

Trois doses ont été comparées : 0, 1 et 10 mg/kg (quantité correspondante d'acide tiludronique/kg). L'acide tiludronique était administré sous forme de solutions aqueuses injectables de telle sorte que le volume injecté par oiseau soit de 1 ml par kilo de poids vif. Les solutions d'acide tiludronique étaient donc concentrées à 0,1 % et 1 % pour les doses de 1 et 10 mg/kg respectivement. Le groupe témoin recevait un placebo correspondant à de l'eau pour préparation injectable. Les injections ont été pratiquées en région sous-cutanée au-dessus du muscle pectoral superficiel.

Les poules étaient hébergées en cages individuelles dans un même bâtiment à température et hygrométrie contrôlées. Chaque animal était identifié par un numéro porté sur une bague fixée à une aile. Elles ont reçu le même aliment standard pour poule pondeuse ad libitum pendant toute la durée de l'étude. L'eau de boisson était également distribuée ad libitum.

L'âge moyen d'entrée en ponte pour les poules de cette souche est de 20 semaines. En conséquence, le délai moyen entre le traitement et l'âge d'entrée en ponte devait être de 3 à 5 semaines. La durée de l'étude a été de 40 semaines. En fin d'étude, les poules étaient âgées de 55 semaines.

La moitié de l'effectif a été sacrifiée à la ponte du premier oeuf en vue de réaliser un examen histomorphométrique pratiqué sur une section décalcifiée de la région proximale d'un des os tarsométatarses pour la mesure des volumes osseux trabéculaire et médullaire.

L'autre moitié de l'effectif a été suivie jusqu'à 55 semaines d'âge (soit après environ 34 semaines de ponte), date à laquelle tous les animaux restants ont été sacrifiés. Le même examen osseux a été conduit chez ces animaux que chez les poules sacrifiées à la ponte du premier oeuf.

Les paramètres osseux ont été comparés par une analyse de variance à 1 facteur (facteur dose).

L'examen histomorphométrique mis en oeuvre permet d'apprécier les changements de structure de l'os.

### 1. Volume de l'os trabéculaire du tarsométatarse

A l'entrée en ponte les volumes osseux trabéculaires apparaissent significativement différents (p = 0,01) entre les groupes (cf tableau 1). Une comparaison des groupes 2 à 2 révèle plus particulièrement une différence significative entre les 2 groupes traités par l'acide tiludronique, l'écart entre les 2 groupes traités mettant en évidence une relation effet-dose. Ainsi, à la dose de 10 mg/kg, la perte osseuse trabéculaire qui précède l'entrée en ponte semble être de moindre importance. Comparativement aux témoins, le gain de volume osseux trabéculaire est de 12 % à la dose de 10 mg/kg.

A 55 semaines d'âge, il existe également une différence très significative (p < 0,01) entre les groupes. Il existe là aussi un effet-dose net, la dose de 10 mg/kg produisant un volume trabéculaire significativement plus élevé que celui des témoins.L'écart entre la dose de 1 mg/kg et les témoins est également important. Il est par ailleurs remarquable de constater qu'à la dose de 1 mg/kg, aucune perte osseuse trabéculaire ne s'est produite au cours de la ponte.

**Tableau 1 :**

| **Volume d'os trabéculaire (exprimé en %) de l'os tarsométatarse chez des poules à l'entrée en ponte et à l'âge de 55 semaines, en fonction de la dose d'acide tiludronique (exprimée en mg/kg)** | | | |
|---|---|---|---|
| Dose d'acide tiludronique (mg/kg) | *0* | *1* | *10* |
| Entrée en ponte | **12,2 ± 3,3** | **10,9 ± 2,9** | **13,7 ± 3,3** |
| A 55 semaines d'âge | **9,8 ± 2,0** | **11,3 ± 2,5** | **12,0 ± 2,0** |
| Variation entre les 2 périodes | **- 20 %** | **+ 4%** | **- 12 %** |

L'os trabéculaire contribue au maintien de l'intégrité structurale de l'os. Les résultats obtenus dans cette étude indiquent donc que l'acide tiludronique a un effet significatif sur la prévention de la perte osseuse trabéculaire qui accompagne le remodelage osseux au cours de la ponte. L'effet est moins marqué sur la prévention de la perte osseuse trabéculaire qui accompagne la formation de l'os médullaire avant l'entrée en ponte, même si le résultat obtenu à la dose de 10 mg/kg tend à indiquer qu'un tel effet préventif peut être atteint en fonction de la dose administrée.

Ces effets sont d'autant plus remarquables que l'acide tiludronique a été administré en injection unique quelques semaines avant l'entrée en ponte, soit plusieurs semaines voire plusieurs mois avant que ces effets n'aient été observés.

La résultante de ces effets est de concourrir au maintien de l'intégrité structural de l'os chez la poule au fur et à mesure que la ponte progresse ; ceci est déterminant pour prévenir l'ostéoporose de la poule.

### 2. Volume de l'os médullaire du tarsométatarse

A l'entrée en ponte les volumes osseux médullaires sont encore très faibles. Ceci traduit la formation récente de l'os médullaire qui débute environ 2 semaines avant l'entrée en ponte ; la variabilité individuelle est particulièrement importante. L'analyse statistique ne révèle aucune différence significative entre les groupes. On note des valeurs moyennes plus faibles chez les animaux traités par l'acide tiludronique que chez les témoins.

A 55 semaines d'âge, les volumes osseux médullaires moyens ne sont pas significativement différents entre les groupes. L'administration d'acide tiludronique n'a donc pas modifié sensiblement le processus de remodelage osseux en cours de ponte. Ce résultat diffère de celui obtenu avec l'acide alendronique qui avait entrainé une diminution significative du volume osseux médullaire chez des animaux traités avant l'entrée en ponte et examinés environ 18 semaines après l'entrée en ponte. La variabilité individuelle est encore importante à cette date. Cependant, l'intensité du remodelage osseux médullaire en cours de ponte apparaît différente entre les animaux témoins et les animaux traités par l'acide tiludronique Alors que chez les témoins, le volume osseux médullaire a été multiplié en moyenne par un facteur 6 après 34 semaines de ponte, le facteur multiplicatif est de 9 à la dose de 1 mg/kg et de 13 à la dose de 10 mg/kg. A cette dose, le volume osseux médullaire est en moyenne de 20 % plus élevé que celui des animaux témoins.

**Tableau 2 :**

| **Volume d'os médullaire (exprimé en %) de l'os tarsométatarse à l'entrée en ponte et à l'âge de 55 semaines, en fonction de la dose d'acide tiludronique (exprimée en mg/kg)** | | | |
|---|---|---|---|
| Dose d'acide tiludronique (mg/kg) | *0* | *1* | *10* |
| A 55 semaines d'âge | **2,93 ± 2,03** | **2,75** + **2,72** | **3,53 ± 3,04** |
| Variation (coefficient multiplicateur) entre l'entrée en ponte et 55 semaines d'âge | **x 6** | **x 9** | **x 13** |

## Revendications

1. Utilisation d'une substance active choisie parmi l'acide tiludronique, un de ses sels pharmaceutiquement acceptable, un de ses hydrates et leurs mélanges, dans la préparation d'un médicament destiné à prévenir et à traiter l'ostéoporose chez la poule pondeuse.

2. Utilisation selon la revendication 1, **caractérisée en ce que** la prévention et le traitement de l'ostéoporose a lieu sans altération du rendement de production des oeufs.

3. Utilisation selon l'une quelconque des revendications 1 ou 2, **caractérisée en ce que** la prévention et le traitement de l'ostéoporose a lieu sans inhibition du développement de l'os médullaire en cours de ponte.

4. Utilisation selon l'une quelconque des revendications 1 à 3, **caractérisée en ce que** la prévention et le traitement de l'ostéoporose assure l'inhibition de la perte osseuse avant et après l'entrée en ponte.

5. Utilisation selon l'une quelconque des revendications 1 à 4, **caractérisée en ce que** la substance active est un sel de sodium de l'acide tiludronique, de préférence le sel disodique.

6. Utilisation selon l'une quelconque des revendications précédentes, **caractérisée en ce que** le médicament est approprié à une administration orale, parentérale ou nasale.

7. Utilisation selon la revendication 6, **caractérisée en ce que** l'administration est initiée dans les six semaines précédant l'âge moyen d'entrée en ponte.

8. Utilisation selon l'une quelconque des revendications 6 ou 7, **caractérisée en ce que** l'administration est réalisée en une seule fois ou à intervalles réguliers avant l'entrée en ponte.

9. Utilisation selon l'une quelconque des revendications 6 à 8, **caractérisée en ce que** chaque dose administrée comprend de 0,5 à 50 mg par kg de poids corporel de substance active.

## Patentansprüche

1. Verwendung eines Wirkstoffs, der aus Tiludronsäure, einem ihrer pharmazeutisch verträglichen Salze, einem ihrer Hydrate und deren Gemischen ausgewählt ist, zur Herstellung eines Arzneimittels, das zur Prophylaxe und Behandlung der Osteoporose bei Legehennen vorgesehen ist:

2. Verwendung nach Anspruch 1, **dadurch gekennzeichnet, dass** Prophylaxe und Behandlung der Osteoporose ohne Veränderung des Umfangs der Eierproduktion durchgeführt werden.

3. Verwendung nach Anspruch 1 oder 2, **dadurch gekennzeichnet, dass** Prophylaxe und Behandlung der Osteoporose ohne Hemmung der Entwicklung der Markknochen in der Legeperiode durchgeführt werden.

4. Verwendung nach einem der Ansprüche 1 bis 3, **dadurch gekennzeichnet, dass** durch die Prophylaxe und Behandlung der Osteoporose der Knochenschwund vor und nach dem Eintritt der Legeperiode gehemmt wird.

5. Verwendung nach einem der Ansprüche 1 bis 4, **dadurch gekennzeichnet, dass** der Wirkstoff ein Natriumsalz der Tiludronsäure und vorzugsweise das Dinatriumsalz ist.

6. Verwendung nach einem der vorhergehenden Ansprüche, **dadurch gekennzeichnet, dass** das Arzneimittel für eine orale, parenterale oder nasale Verabreichung geeignet ist.

7. Verwendung nach Anspruch 6, **dadurch gekennzeichnet, dass** mit der Verabreichung in den sechs Wochen begonnen wird, die dem mittleren Alter des Eintritts der Legeperiode vorhergehen.

8. Verwendung nach Anspruch 6 oder 7, **dadurch gekennzeichnet, dass** die Verabreichung auf einmal oder in regelmäßigen Abständen vor Eintritt der Legeperiode erfolgt.

9. Verwendung nach einem der Ansprüche 6 bis 8, **dadurch gekennzeichnet, dass** jede verabreichte Dosis 0,5 bis 50 mg Wirkstoff pro kg Körpergewicht umfasst.

## Claims

1. Use of an active substance selected from among tiludronic acid, one of the pharmaceutically acceptable salts thereof, one of the hydrates thereof and mixtures thereof in the preparation of a medicine intended to prevent and treat osteoporosis in laying hens.

2. Use according to claim 1, **characterised in that** the prevention and treatment of osteoporosis takes place without impairing egg production yield.

3. Use according to either one of claims 1 or 2, **characterised in that** the prevention and treatment of osteoporosis takes place without inhibiting the development of medullary bone during laying.

4. Use according to any one of claims 1 to 3, **characterised in that** the prevention and treatment of osteoporosis ensures the inhibition of bone loss before and after the start of laying.

5. Use according to any one of claims 1 to 4, **characterised in that** the active substance is a sodium salt of tiludronic acid, preferably the disodium salt.

6. Use according to any one of the preceding claims, **characterised in that** the medicine is suitable for oral, parenteral or nasal administration.

7. Use according to claim 6, **characterised in that** administration is initiated within the six weeks preceding the average age at which laying starts.

8. Use according to either one of claims 6 or 7, **characterised in that** administration is performed on a single occasion or at regular intervals before the start of laying.

9. Use according to any one of claims 6 to 8, **characterised in that** each dose administered comprises 0.5 to 50 mg of active substance per kg of body weight.
